# EUROPEAN PATENT APPLICATION

(11) **EP 4 483 813 A1**
(43) Date of publication of application: **01.01.2025**
(21) Application number: 23182235.4
(22) Date of filing: 28.06.2023
(51) Int. Cl.: A61B 6/04, A61B 6/00, A61D 3/00

(54) **ANIMAL HEATER PAD**

(71) Applicant: Molecubes, 9052 Gent (BE)
(72) Inventor: DEPREZ, Karel, Gent (BE); De Cauwer, Simon, 9031 Drongen (BE)
(74) Representative: Winger

(57) **Abstract**

A specimen support system (100) for supporting a small animal during pre-clinical imaging. The specimen support system (100) comprises an animal bed (110) for supporting the small animal, and a heating system linked to the animal bed (110). The heating system comprises a heat pad for influencing the temperature of the small animal when supported on the animal bed (110), the heat pad comprising a layer of graphite (120). The heating system is adapted for controlling an energy supplied to the layer of graphite (120) thus influencing the temperature of the small animal.

## Description

### Field of the invention

The present invention relates to heating methods and systems for use with animal beds in imaging applications.

### Background of the invention

For preclinical imaging as well as for testing purposes, animals, including rodents, are often subjected to imaging techniques such as for example single photon emission computed tomography SPECT, positron emission tomography (PET), computed tomography (CT), computer aided tomography (CAT), X-ray or magnetic resonance imaging techniques such as nuclear magnetic resonance imaging (MRI), to obtain real-time or photographic images, e.g. without harming the animals. Since animals should remain motionless during the imaging, e.g. the scanning process, anaesthesia is often administered. However, anesthetized animals, in particular rodents, can struggle to maintain their body temperature within the desired range, which can cause additional stress to the animals.

In view thereof, heating systems are typically used to effectively regulate the body temperature of the immobilized animals throughout the anaesthesia and the imaging process. In order not to deteriorate the quality of the imaging, typically the heating systems and methods used need to be compatible with the respective requirements of the imaging techniques.

Some existing heating systems for animal beds are based on hot air heating. Such heating systems typically require a pneumatic coupling (air coupling) between the heating system and the bed. Furthermore, they often are large in size and may require a heat exchanger. An example of a hot air heating system for an animal bed is described in US patent 9,408,682 B2, describing a system with an animal bed, a heater positioned outside the animal bed, the heater on one side being coupled to an air pump for receiving unheated air, and on the other hand being coupled to the animal bed through channels for transporting the heated air to the animal bed.

There is nevertheless a room for improvement of the heating systems and methods used in animal beds.

### Summary of the invention

It is an aim of embodiments of the present invention to provide good heating systems and corresponding methods for heating animal beds used for pre-clinical imaging of animals.

It is an advantage of embodiments according to the present invention that the heating system and corresponding method is compatible with pre-clinical imaging techniques for animals, such as for example computed tomography (CT), X-ray imaging, positron emission tomography (PET), single photon emission computed tomography (SPECT), ultrasound imaging, optical imaging, magnetic resonance imaging (MRI), etc.

It is an advantage of embodiments according to the present invention that the heating system and corresponding method avoids to a large extend the use of metals, since metals typically induce artefacts in the imaging.

It is an advantage of embodiments according to the present invention that the heating system does not make use of dense materials, thus avoiding heavily attenuation of e.g. X-rays.

It is an advantage of embodiments according to the present invention that the heating system is compact, allowing easier integration in the animal beds, where little space is available.

The above-mentioned aim is achieved by a system and method as described below.

In a first aspect, the present invention relates to a specimen support system for supporting a small animal during pre-clinical imaging, the specimen support system comprising an animal bed for supporting the small animal and a heating system linked to the animal bed. The heating system comprises a heat pad for influencing the temperature of the small animal when supported on the animal bed. The heat pad comprises a layer of graphite. The heating system is adapted for controlling an energy supplied to the layer of graphite thus influencing the temperature of the small animal. According to some embodiments of the present invention, the energy supplied to the layer of graphite may be electrical energy, e.g. electrical current, which is converted in the layer of graphite to thermal energy for influencing the temperature of the small animal. According to some embodiments of the present invention, the energy supplied to the layer of graphite may be thermal energy, e.g. heat, which is thermally conducted by the layer of graphite and transferred to the surrounding for influencing the temperature of the small animal.

The layer of graphite may be a thin sheet. Where in embodiments reference is made to a thin sheet, reference is made to a structure wherein the thickness is substantially smaller, e.g. at least 10 times smaller, than the dimensions in a width and a length direction in which the structure is extending.

Where in embodiments reference is made to thickness, reference is made to the dimension of the structure along the local normal to the structure.

The thin sheet may for example have an average thickness between 10µm and 1000µm. It is an advantage of embodiments of the present invention that the thickness of the heating system is small and the heating system thus is compact, since the available amount of space in the animal bed is limited.

The heating system may be configured for inducing electrical current in the layer of graphite for controlling the energy supplied to the layer of graphite. It is an advantage of embodiments of the present invention that the good electrical properties (low resistivity) of graphite layers can be used for inducing heating of the graphite layer and thus influencing of the temperature of the small animal positioned on the animal bed.

The heating system may comprise an adjustable power supply and wherein the heat pad is electrically connected to the power supply via at least two electrical connections. Alternatively, the heat pad may be provided with electrical connections and the power supply may be external to the specimen support system but connectable to the electrical connections of the heat pad. In yet other embodiments, electrical current may be induced in a different way in the layer of graphite, e.g. inductively or alike.

The heat pad may extend along the length of the animal bed and may make at least one turn. The at least two electrical connections of the heat pad to the power supply may be positioned at the same side of the heat pad. The electrical power supply, the electrical connections and the heat pad may be arranged to form a closed electrical circuit. It is an advantage of embodiments of the present invention that the electrical connections of the heat pad are positioned at one side of the heat pad, since electrical connections which are typically made of metal components induce artefacts in some imaging techniques. When the electrical connections are at the same side of the heat pad, they can be positioned more easily outside the imaging part of the bed.

The heat pad may, at the position of the electrical connections, be wider than the average width of the heat pad along the length of the heat pad. It is an advantage of embodiments of the present invention that the heat pad at the position of the electrical connections is wider than average, as it allows to reduce the heat generation due to lower electrical resistance in that zone and as it allows for an easier electrical connection. It is an advantage of embodiments of the present invention that the thickness and surface area of the heat pad can be amended, designed and/or optimized for shaping the heat dissipation of the heat pad's surface and for shaping the electrical characteristics (e.g. resitivity).

The pre-clinical imaging modality may be at least one of CT, SPECT, PET, ultrasound, magnetic resonance or optical imaging.

The support may be adapted for supporting rodents.

The heat pad may be covered on at least one side, preferably on both sides, with a shell material. The shell material may be a carbon shell. It is an advantage of embodiments using a carbon shell material that it is light in weight, is easy to clean, while the heating system remains compact. The shell material advantageously may provide a mechanical protection for the thermal pad. Furthermore, it may render the cradle easier to clean. Carbon materials such as for example - but not limited to - carbon fibre tubes are typically relatively low in cost and have a good strength and stiffness. Furthermore, such materials are typically transparent for radiation used in at least some types of imaging and do not introduce artefacts on the corresponding acquired images. In other examples, the shell material may comprise other materials such as plastics or ceramics, or may combine different materials at different positions along the shell or on top of each other.

The layer of graphite may be covered on one or both sides with a layer comprising electrically insulating material. The electrically insulating material may be capton. It is an advantage of embodiments of the present invention that the layer of graphite may be electrically isolated from potentially conductive materials in the shell or in other components of the specimen support system.

The heating system furthermore or alternatively may comprise a thermal power source in thermal connection with the heat pad for controlling thermal energy supplied to the layer of graphite by controlling the temperature of the thermal power source. The thermal power source may be a power resistor.

According to one aspect, the present invention also relates to an imaging system for pre-clinical imaging of small animals, the imaging system comprising a specimen support system according to one of the embodiments as described above, and an imager for pre-clinical imaging of a small animal positioned on the specimen support system.

Specific and preferred aspects of the invention have been included in the attached independent and dependent claims. Features of the dependent claims can be combined with features of the independent claims and with features of further dependent claims such as indicated and not only as expressly brought forward in the claims.

To summarise the invention and the advantages achieved compared with the state of the art, certain aims and advantages of the invention were described above. It may be understood, of course, that not necessarily all these aims or advantages can be achieved by each specific embodiment of the invention. So, for example, it will be obvious to a person skilled in the art that the invention may be embodied or carried out in a manner which reaches or optimises one advantage or a group of advantages as presented herein, without thereby necessarily achieving other aims or advantages which may have been presented or suggested herein.

The above and other aspects of the invention will be apparent and clarified by reference to the embodiment(s) described below.

### Short description of the drawings

The invention will now be further described, by means of example, with reference to the associated figures wherein:
FIG. 1 shows a specimen support system according to an embodiment of the present invention.
FIG. 2 shows the heat distribution using a specimen support system according to an embodiment of the present invention.
FIG. 3 shows a schematic illustration of an imaging system according to an embodiment of the present invention.

The figures are only schematic and not restrictive. It is possible that the dimensions of some components have been exaggerated and have not been represented to scale in the figures for illustrative purposes. Dimensions and relative dimensions do not necessarily correspond with actual embodiments of the invention.

Reference numbers used in the claims cannot be interpreted to restrict the scope of protection.

### Detailed description of illustrative embodiments

While the invention is illustrated and described in detail in the drawings and accompanying description, such illustration and description are to be considered illustrative or exemplary and not restrictive. The invention is not limited to the disclosed embodiments.

Other variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

The following description details certain embodiments of the invention. It will be appreciated, however, that no matter how detailed it appears in text, the invention may be practiced in many ways, and is therefore not limited to the embodiments disclosed. It should be noted that the use of particular terminology when describing certain features or aspects of the invention should not be taken to imply that the terminology is being re-defined herein to be restricted to include any specific characteristics of the features or aspects of the invention with which that terminology is associated.

In a first aspect, the present invention relates to a specimen support system for supporting a small animal during pre-clinical imaging. The specimen support system comprises an animal bed for supporting the small animal and a heating system linked to the animal bed. Such an animal bed may be part of a larger system, e.g. an imaging system for pre-clinical imaging of a small animal. Where in embodiments of the present invention reference is made to a small animal, the latter typically may refer to rodents, such as for example mice, rats, squirrels, prairie dogs, porcupines, beavers, guinea pigs, hamsters, etc., but may also include other animals such as for example rabbits, hares, pikas or more generally the group of Lagomorpha or for example birds, domestic animals, farm animals, etc. According to embodiments of the present invention, the specimen support system also comprises a heating system comprising a heat pad for influencing the temperature of the small animal when supported on the animal bed. The latter allows for influencing, e.g. controlling, the temperature of the animal positioned on the animal bed. Since e.g. during pre-clinical imaging, animals often are anesthetized and may struggle with keeping their body temperature, the latter allows for more animal-friendly diagnostics and treatments. According to embodiments of the present invention, the heat pad comprises a layer of graphite. The heating system is adapted for controlling an energy supplied to the layer of graphite thus influencing the temperature of the small animal. Use of a layer of graphite allows for good thermal transfer between the heating system and the remainder of the animal bed and thus to the animal positioned thereon.

According to some embodiments of the present invention, the energy supplied to the layer of graphite may be electrical energy, e.g. electrical current, which is converted in the layer of graphite to thermal energy for influencing the temperature of the small animal. According to some embodiments of the present invention, the energy supplied to the layer of graphite may be thermal energy, e.g. heat, which is thermally conducted by the layer of graphite and transferred to the surrounding for influencing the temperature of the small animal.

In embodiments of the present invention, the heating system thus has a heat pad comprising a graphite layer which may be a thin sheet. The layer may be self-supportive or may be positioned on a substrate. The layer may have an average thickness between 10µm and 1000µm. It is an advantage of embodiments of the present invention that the thickness of the heating system is small and the heating system thus is compact, since the available amount of space in the animal bed is limited. The layer of graphite may have a shape comprising at least one turn, e.g. have a U shape, although embodiments are not limited thereto. It may be shaped according to a specific pattern, corresponding with positions where heat needs to be transferred to the animal bed. In some embodiments, the layer is a self-supportive layer that can be easily cut to a predetermined shape, where required even complex shapes, thus allowing to provide heat at the appropriate positions. The shape could be selected to interfere as little as possible with the imaging system and the imaging technique, so as to avoid artefacts in the images obtained.

In embodiments wherein the energy supplied to the layer of graphite is electrical energy that is converted by the layer of graphite itself through resistive heating, the shape of the layer of graphite may be such it forms part of a closed electrical circuit. In advantageous embodiments, the local thickness and width of the layer of graphite may be selected so as to have an appropriate resistivity distribution and hence an appropriate distribution of heat dissipation in the layer through resistive heating. The most appropriate distribution may be selected as function of the specimen support system. By locally selecting the thickness and the width of the layer, the amount of heat dissipated in the layer can be selected. In other words, by changing the width and the length of the graphite pattern, the heat, power and current characteristics in the graphite layer can be tuned.

In some embodiments, e.g. when a thermal power source is used and the energy transfer to the layer of graphite, the layer of graphite does not need to form a closed circuit.

It is an advantage of embodiments of the present invention that the graphite layer does not comprise metal particles and therefore does not interfere substantially with most imaging techniques. It also is an advantage of embodiments of the present invention that the layer of graphite typically is a good and uniform heat spreader.

The layer of graphite may be flexible and/or foldable. Use may be made of this flexibility at the position where the layer of graphite is contacted to the energy source. In case of electrical energy supplied to the layer of graphite, electrical contact may be established by fixing electrical wires to the layer of graphite, e.g. by bonding, glueing e.g. using conductive glue, soldering, wrapping the wires in sheet shaped portions of the layer of graphite, or alike. Similarly, in case of thermal energy supplied to the layer of graphite, thermal conductors, such as metal portions, may be fixed to the layer of graphite in any suitable manner allowing good thermal contact.

The heating system also typically may comprise a power supply (typically adjustable). In case of electrical energy being supplied to the layer of graphite, such a power supply may be any suitable electrical power supply for providing electrical current to the layer of graphite. Such a source may for example be implemented in a printed circuit board (PCB), although embodiments are not limited thereto. Whereas it is advantageous that the electrical power supply is integrated in the specimen support system, it may also be a component that can be positioned further away. The electrical power supply may be adapted for providing appropriate current signals to the layer of graphite layer. The latter may e.g. be performed under command of a controller, which may also be a separate component or a component integrated on the printed circuit board. In some embodiments, the electrical power supply may be configured for providing modulated current signals to the graphite layer. Such modulated current signals may e.g. be pulse width modulated current signals, since these easily allow to control the heat developed in the graphite layer.

Alternatively, or in addition thereto, the heating system also may comprise a thermal power source and the layer of graphite may be heated through thermal conductivity. Examples of thermal power sources are for example power resistors, although other power sources also can be used. When making use of thermal power sources, in order to protect other components of the system, thermally insulating materials may be used to shield the thermal power sources from the other components.

The specimen support system may optionally also comprise a holding component for mechanically holding the power source, such as for example a bracket. In some embodiments, such a bracket may be a bracket for holding a printed circuit board. The holding component also may be configured to hold different components of the specimen support system, optionally with thermal insulation between the components.

In some embodiments, the specimen support system may further comprise a monitoring and/or controlling system for monitoring parameters, such as for example vital parameters of the animal such as breathing, heart rate, ECG, temperature, etc. and/or for controlling other elements of the specimen support system. Such monitoring and/or controlling may be integrated together with the electrical supply source as described above, e.g. on a same printed circuit board. Alternatively, such a monitoring and/or controlling system may be implemented on a separate platform, e.g. on a separate printed circuit board.

The animal bed may comprise a plate or shell shaped component which may act as surface to which the animal can be positioned. The graphite layer may advantageously be positioned in contact with the plate or shell shaped component, to allow for conductive heat transfer to the plate or shell shaped component and thus to a small animal positioned thereon. In some embodiments, the animal bed may comprise two plate or shell shaped components between which the graphite layer may be positioned. The plate or shell shaped components may be made of a material that is easy to clean, such as for example carbon fiber. Moreover, advantageously a material is selected for the plate or shell shaped component(s) that does not or only little interfere with the radiation used for imaging, so as to avoid artefacts.

A system according to embodiments of the present invention also may comprise a connection piece for connecting the specimen support system to the remainder of the imaging system in which the specimen support system is used. Such a connection piece may for example be a metal block with particular fixing means for fixing it, but also other types of materials may be used.

In some embodiments, the system also may comprise anaesthesia tubing, an anaesthesia input and scavenging, etc. Furthermore, other components that often may be present in animal beds according to the current state of the art also may be present, as will be appreciated by the person skilled in the art.

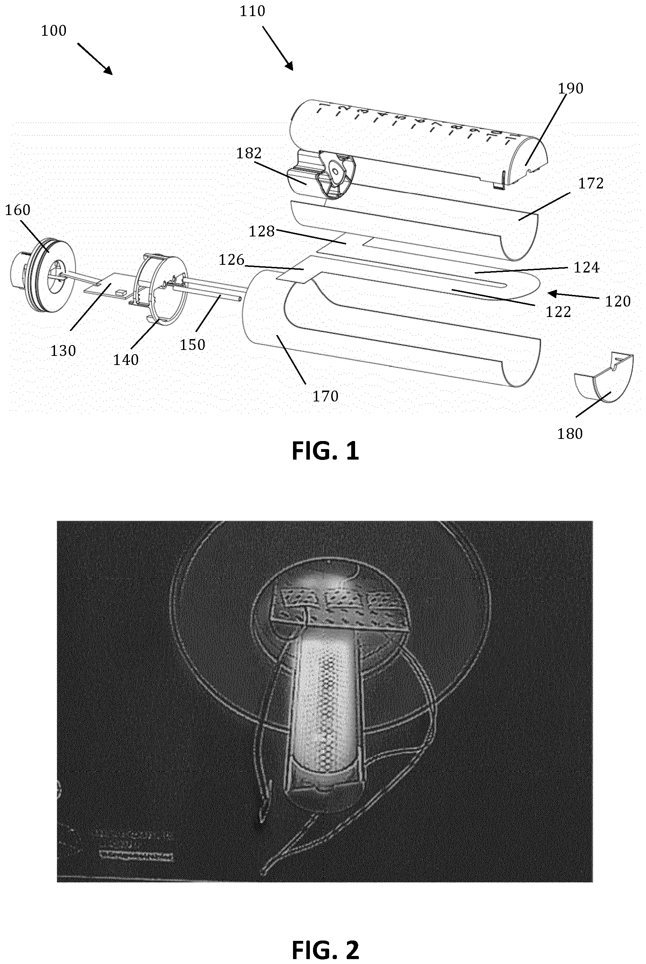

By way of illustration, embodiments of the present invention not being limited, an exemplary specimen support system 100 is shown in FIG. 1. The specimen support system 100 comprises the animal bed 110 and the layer of graphite 120. In the example shown, the graphite layer 120 has a specifically patterned shape. The layer 120 is U-shaped and has two legs 122, 124 with each a wide portion 126, 128, the wide portions 126, 128 both being positioned at the same side. Electrical connections between a printed circuit board 130 and the graphite layer 120 in the present example are made using electrical wires - not shown - between the two wide portions 126, 128 of the graphite layer 120 and the printed circuit board 130. By using wide portions 126, 128 of the graphite layer 120, the amount of heat dissipation that is occurring at this position, i.e. close to the printed circuit board, due to resistive heating in the graphite layer 120, is limited. As a consequence, the heat dissipated in the smaller portions of the legs 122, 124 will be higher. In the present example, the power supply 130 on the printed circuit board is an electronic board that at the same time comprises the electrical power source for supplying electrical current to the graphite layer 120, and other electrical components of the system supporting like e.g. monitoring of vital signs of the animal, such as for example the breathing, an ECG, the temperature, and/or e.g. controlling of other features of the system that need to be electronically controlled. The specimen support system shown in FIG. 1 also illustrates a holding bracket 140 for holding the electronic board (PCB), anaesthesia tubing 150, a connection piece 160 being a metal bed block, an outer carbon fiber shell 170 and an inner carbon fiber shell 172 in between which the graphite layer 120 is positioned, mounting elements 180, 182 and an upper cover 190.

Further by way of illustration, the thermal heating of the specimen support system using a particular embodiment of the present invention is shown in FIG. 2. FIG. 2 shows a thermal (infrared) image of the specimen support system. It can be seen that an accurate spreading of the heat is obtained. The latter confirms that the systems allow for accurate heating of animals positioned on the animal bed.

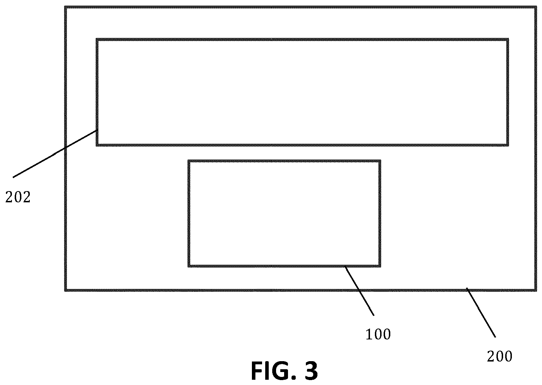

In a further aspect, the present invention relates to an imaging system for pre-clinical imaging of small animals. Such an imaging system may for example make use of e.g. computed tomography (CT), X-ray imaging, positron emission tomography (PET), single photon emission computed tomography (SPECT), ultrasound imaging, optical imaging, magnetic resonance imaging (MRI), etc. The imaging system typically comprises an imager suitable for pre-clinical imaging - although imaging at other moments in time during diagnostics, treatment or research on small animals also may be performed - as well as a specimen support system according to the first aspect of the present invention. Since components of the imager typically need to surround the small animals positioned on the specimen support system, it is an advantage that the specimen support system according to embodiments of the present invention can be compact. The imager may be of a scanning device or based on other imaging methods. It is to be noted that components of such imagers, including but not limited to irradiation sources and detectors, typically are known to the person skilled in the art and therefore are not discussed here. By way of illustration, a schematic view of such an imaging system 200, including an imager 202 and a specimen support system 100 is shown in FIG. 3.

### Reference numbers

- 100: specimen support system
- 110: animal bed
- 120: graphite layer
- 122, 124: legs
- 126, 128: wide portions
- 130: power supply, printed circuit board
- 140: holding bracket
- 150: anaesthesia tubing
- 160: connection piece
- 170: outer carbon shell
- 172: inner carbon shell
- 180, 182: mounting elements
- 190: upper cover
- 200: imaging system
- 202: imager

## Claims

1. A specimen support system (100) for supporting a small animal during pre-clinical imaging, the specimen support system (100) comprising
- an animal bed (110) for supporting the small animal, and
- a heating system linked to the animal bed (110),
the heating system (110) comprising a heat pad for influencing the temperature of the small animal when supported on the animal bed (110), the heat pad comprising a layer of graphite (120), and
the heating system being adapted for controlling an energy supplied to the layer of graphite (120) thus influencing the temperature of the small animal.

2. A specimen support system (100) according to claim 1, wherein the layer of graphite (120) is a thin sheet layer having an average thickness between 10µm and 1000pm.

3. A specimen support system (100) according to any of the previous claims, wherein the heating system is configured for inducing electrical current in the layer of graphite (120) for controlling the energy supplied to the layer of graphite (120).

4. A specimen support system (100) according to any of the previous claims, wherein the heating system comprises a power supply (130) and wherein the heat pad is electrically connected to the power supply (130) via at least two electrical connections.

5. A specimen support system (100) according to the previous claim, wherein the heat pad extends along the length of the animal bed (110) and makes at least one turn, and wherein the at least two electrical connections of the heat pad to the power supply (130) are positioned at a same side of the heat pad.

6. A specimen support system (100) according to any of claims 4 to 5, wherein the electrical power supply (130), the electrical connections and the heat pad form a closed electrical circuit.

7. A specimen support system (100) according to any of claims 4 to 6, wherein the heat pad at the position of the electrical connections is wider than the average width of the heat pad along the length of the heat pad.

8. A specimen support system (100) according to any of the previous claims, wherein the pre-clinical imaging modality is at least one of CT, SPECT, PET, ultrasound, magnetic resonance or optical imaging.

9. A specimen support system (100) according to any of the previous claims, wherein the animal bed (110) is adapted for supporting rodents.

10. A specimen support system (100) according to any of the previous claims, wherein the heat pad is covered on at least one side, preferably on both sides, with a shell material.

11. A specimen support system (100) according to any of the previous claims, wherein the layer of graphite (120) is covered on one or both sides with a layer comprising electrically insulating material.

12. A specimen support system (100) according to the previous claim, wherein the electrically insulating material comprises capton.

13. A specimen support system (100) according to any of the previous claims, wherein the heating system furthermore comprises a thermal power source in thermal connection with the heat pad for controlling the energy supplied to the layer of graphite (120) by controlling the temperature of the thermal power source.

14. A specimen support system (100) according to claim 13, wherein the thermal power source is a power resistor.

15. An imaging system (200) for pre-clinical imaging of small animals, the imaging system (200) comprising a specimen support system (100) according to any of the previous claims, and an imager (202) for pre-clinical imaging of a small animal positioned on the specimen support system (100).
